# EUROPEAN PATENT APPLICATION

(11) **EP 2 135 571 A1**
(43) Date of publication of application: **23.12.2009**
(21) Application number: 09251400.9
(22) Date of filing: 27.05.2009
(51) Int. Cl.: A61B 17/34

(54) **Surgical seal with variable diameter**

(30) Priority: 17.06.2008 US 73177 P; 30.04.2009 US 432928
(71) Applicant: Tyco Healthcare Group LP, North Haven, CT 06473 (US)
(72) Inventor: Zemlock, Michael A., Prospect, CT 06712 (US)
(74) Representative: Soames, Candida Jane

(57) **Abstract**

A surgical access assembly dimensioned for positioning within a patient's tissue includes a seal member having an internal channel defined therein for the reception of a closure member, and an aperture extending through the seal member that is configured to removably receive a surgical object upon its insertion into the surgical access assembly. The closure member is selectively actuable to transition the seal member from a first condition, in which the aperture includes a first diameter, to a second condition, in which the aperture includes a second diameter. The second diameter is less than the first diameter and substantially approximates an outer dimension of the surgical object such that a substantially fluid-tight seal is formed therewith.

## Description

### CROSS REFERENCE TO RELATED APPLICATION

The present application claims the benefit of and priority to U.S. Provisional Application Serial No. 61/073,177 filed on June 17, 2008, the entire contents of which are incorporated herein by reference.

### BACKGROUND

### 1. Technical Field

The present disclosure relates to a surgical access assembly which is removably insertable into a patient's tissue. The access assembly includes a seal adapted for the reception of a surgical object and the formation of a substantially fluid-tight seal therewith.

### 2. Background of the Related Art

Many surgical procedures are performed through access assemblies, e.g., trocar and cannula assemblies. These assemblies incorporate narrow tubes or cannulae percutaneously inserted into a patient's body, through which one or more surgical objects may be introduced and manipulated during the course of the procedure. Generally, such procedures are referred to as "endoscopic", unless performed on the patient's abdomen, in which case the procedure is referred to as "laparoscopic". Throughout the present disclosure, the term "minimally invasive" should be understood to encompass both endoscopic and laparoscopic procedures.

Generally, during minimally invasive procedures, prior to the introduction of a surgical object into the patient's body, insufflation gas is used to enlarge the area surrounding the target surgical site to create a larger, more accessible work space. The maintenance of a substantially fluid-tight seal along the central opening of the access device in the presence of the surgical object is therefore desirable to curtail the escape of insufflation gas and the deflation or collapse of the enlarged work space. To this end, surgical access devices generally incorporate a seal through which the surgical object is inserted.

During the course of a minimally invasive procedure, it is often necessary for a clinician to employ and interchange surgical objects of various sizes, e.g., surgical objects that vary in their outer peripheral dimensions. While many varieties of seals are known in the art, there exists a continuing need for a seal that can accommodate a variety of differently-sized surgical objects in substantially sealed relation to thereby maintain the integrity of an insufflated workspace.

### SUMMARY

In one aspect of the present disclosure a surgical access assembly is disclosed. The surgical access assembly includes an access member dimensioned for positioning in a patient's tissue and defining a passageway that extends longitudinally therethrough that is configured to removably receive a surgical object. The surgical access assembly comprises a seal member including an internal channel formed therein, and at least one closure member at least partially disposed within the internal channel. In alternate embodiments, the at least one closure member extends from the seal member through an egress formed in its proximal surface, periphery, or any other suitable location. The seal member has an aperture that extends therethrough, and is selectively adaptable to transition between first and second conditions upon actuation of the at least one closure member. In the first condition, the aperture includes a first diameter that allows for the insertion of a surgical object, and in the second condition, the aperture includes a second smaller diameter that substantially approximates an outer dimension of the surgical object such that a substantially fluid-tight seal is formed therewith.

In one embodiment, the seal member is formed of a material that is at least semi-resilient in nature such that the aperture of the seal member is normally biased towards the first condition. Alternatively, or additionally, the aperture of the seal member may be biased towards the first condition through the incorporation of at least one biasing member. The biasing member may be disposed within the internal channel, and may define a passage therethrough configured to receive the at least one closure member. In one embodiment, the biasing member is a spring.

The surgical access assembly further includes a housing at a proximal end thereof, from which the access member extends distally. The housing may include at least one opening configured to allow the at least one closure member to pass therethrough such that at least a portion of the at least one closure member is disposed externally of the surgical access assembly.

The surgical access assembly may also include attachment structure configured to releasably secure the at least one closure member when the seal member is in the second condition.

In another aspect of the present disclosure, the at least one closure member is secured to a tensioning mechanism. The tensioning mechanism includes a selectively actuable motor, to which the at least one closure member is operatively secured. The motor may be operatively connected to a spool member, which may in turn be secured to the at least one closure member. The motor is adapted to move the spool member through a plurality of positions to thereby wind and unwind the at least one closure member about the spool member such that the tension applied to the at least one closure member may be respectively increased and decreased.

The tensioning mechanism may also include a sensor operably coupled to the housing of the surgical access assembly. The sensor is adapted to detect at least one attribute of the surgical object, including but not being limited to an outer dimension, color, electrical impedance, or magnetic impedance thereof, upon introduction of the surgical object into the surgical access assembly. In response to the detection of the surgical object, the sensor generates a first electrical signal.

These and other features of the seal disclosed herein will become more readily apparent to those skilled in the art from the following detailed description of various embodiments of the present disclosure.

The invention may be described by reference to the following numbered paragraphs:-

A surgical access assembly, which comprises:
an access member dimensioned for positioning with a patient's tissue and defining a passageway extending longitudinally therethrough configured to removably receive a surgical object;
a seal member including an internal channel defined therein and having an aperture extending therethrough, the seal member being selectively adaptable to transition between a first condition wherein the aperture includes a first diameter and a second condition wherein the aperture includes a second diameter that is smaller than the first diameter; and
at least one closure member at least partially disposed within the internal channel which is actuatable to transition the diameter of the seal member from the first condition to the second condition upon actuation thereof.

The surgical access assembly of claim [0014], wherein the aperture defines a first transverse dimension when the seal member is in the first condition allowing for the insertion of a surgical object and the aperture defines a second transverse dimension when the closure member is actuated to move the seal member to the second condition, the second transverse dimension substantially approximating an outer dimension of the surgical object such that a substantially fluid-tight seal is formed therewith.

The surgical access assembly of claim [0015], wherein the seal member is formed of a material that is at least semi-resilient such that the aperture of the seal member is normally biased towards the first condition.

The surgical access assembly of claim [0014], further including at least one biasing member disposed within the seal member, the biasing member being adapted to normally bias the aperture of the seal member towards the first condition.

The surgical access assembly of [0017], wherein the at least one biasing member is disposed within the internal channel.

The surgical access assembly of [0018], wherein the at least one biasing member defines a passage therethrough configured to receive the at least one closure member.

The surgical access assembly of [0019], wherein the biasing member is a spring.

The surgical access assembly of [0014], wherein the at least one closure member extends from the seal member through an egress formed therein.

The surgical access assembly of [0021], wherein the egress is formed in a proximal surface of the seal member.

The surgical access assembly of [0021], wherein the egress is formed in a periphery of the seal member.

The surgical access assembly of [0021], further including a housing at a proximal end thereof, the access member extending distally from the housing.

The surgical access assembly of [0024], wherein the housing includes at least one opening configured to allow the at least one closure member to pass therethrough such that at least a portion of the at least one closure member is disposed externally of the surgical access assembly.

The surgical access assembly of [0025], further including attachment structure configured to releasably secure the at least one closure member when the seal member is in the second condition.

The surgical access assembly of [0025], wherein the at least one closure member is secured to a tensioning mechanism.

The surgical access assembly of [0027], wherein the tensioning mechanism includes a motor operatively secured to the at least one closure member, the motor being selectively actuable.

The surgical access assembly of [0028], wherein the at least one closure member is secured to a spool member operatively connected to the motor, the motor being adapted to reposition the spool member to thereby wind and unwind the at least one closure member about the spool member such that the tension applied to the least one closure member may be respectively increased and decreased.

The surgical access assembly of [0029], wherein the tensioning mechanism includes a sensor operably coupled to the housing, the sensor being adapted to detect at least one attribute of the surgical object upon the introduction thereof into the surgical access assembly and generate a first electrical signal in response thereto to actuate the closure member.

The surgical access assembly of [0030], wherein the at least one attribute of the surgical object is selected from the group consisting of an outer dimension, color, electrical impedance, and magnetic impedance.

### BRIEF DESCRIPTION OF THE DRAWINGS

Various embodiments of the present disclosure are described herein below with references to the drawings, wherein:

FIG. 1 is a side, schematic view of a surgical access assembly with a seal disposed therein in accordance with the principles of the present disclosure;

FIG. 2A is a top, schematic view of the seal of FIG. 1 shown in a first condition and including a closure member partially disposed within an internal channel formed in the seal, wherein the closure member includes a first end that is fixedly secured within the internal channel and a second end that extends outwardly therefrom;

FIG. 2B is a side, cross-sectional view of the seal of FIGS. 2A;

FIG. 2C is a top, perspective view of an alternate embodiment of the seal of FIG. 2A;

FIG. 3 is a top, perspective view of the seal of FIG. 2A shown in a second condition with a surgical object inserted therethrough;

FIG. 4A is a top, schematic view of another embodiment of the seal of FIG. 2A shown in a first condition and including a biasing member disposed within the internal channel;

FIG. 4B is a side, cross-sectional view of the seal of FIG. 4A;

FIG. 4C is a top, schematic view of the seal of FIG. 4A shown in a second condition;

FIG. 4D is a top, schematic view of another embodiment of the seal of FIG. 4A, wherein the biasing member is disposed between the internal channel and an aperture extending through the seal;

FIG. 4E is a top, schematic view of an alternate embodiment of the seal of FIG. 4A, wherein the biasing member is substantially tubular in configuration;

FIG. 4F is a top, schematic view of one embodiment of the seal of FIG. 4E, wherein the biasing member is disposed between the internal channel and a periphery of the seal;

FIG. 5 is a top, schematic view of yet another embodiment of the seal of FIG. 2A, wherein the first and second ends of the closure member each extend outwardly from the seal;

FIG. 6 is a side, schematic view of an alternate embodiment of the surgical access assembly of FIG. 1, further including a tensioning mechanism; and

FIG. 7 is a side, schematic view of the surgical access assembly of FIG. 6.

### DETAILED DESCRIPTION

In the drawings and in the description which follows, in which like references numerals identify similar or identical elements, the term "proximal" will refer to the end of the apparatus which is closest to the clinician, while the term "distal" will refer to the end which is furthest from the clinician, as is traditional and known in the art. Additionally, use of the term "surgical object" herein below should be understood to include any surgical object or instrument that may be employed during the course of surgical procedure, including but not being limited to an obturator, a surgical stapling device, or the like.

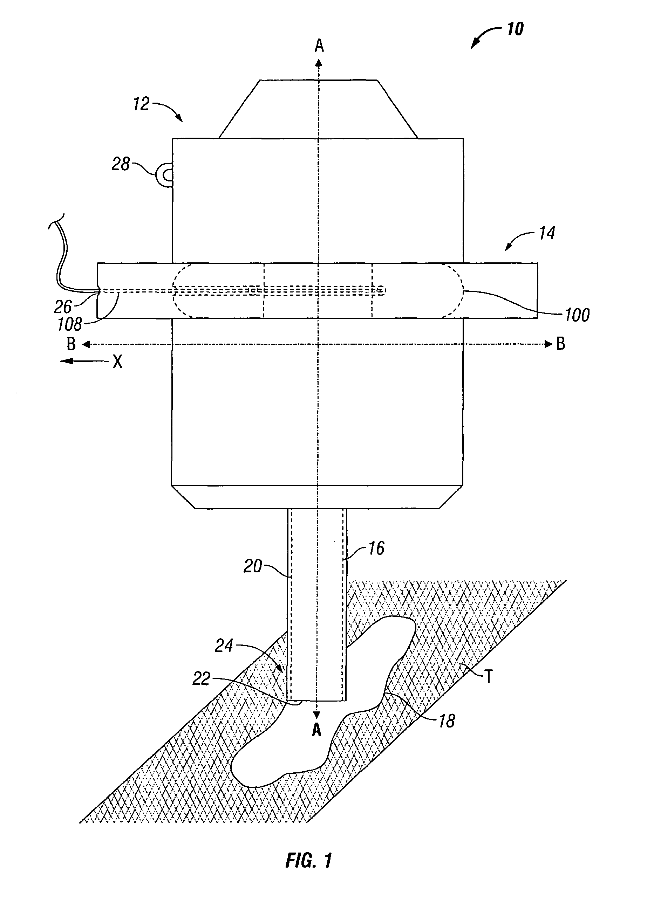

**FIG. 1** illustrates a surgical access assembly **10,** which is the subject of the present disclosure. At a proximal end **12** thereof, surgical access assembly **10** includes a housing **14** configured to accommodate a seal member **100,** and may be any structure suitable for this intended purpose. Further information regarding seal housing **14** may be obtained through reference to commonly owned U.S Patent No. 7,169,130 to Exline et al**.,** the entire contents of which are incorporated by reference.

Extending distally from housing **14** is an access member **16** that is dimensioned for positioning with a percutaneous access point **18,** either pre-existing or created by a clinician, formed in a patient's tissue **"T".** Access member **16** defines a passageway **20** that extends longitudinally therethrough along a longitudinal axis **"A-A"** and is configured for the internal receipt of one or more surgical objects (not shown). Access member **16** defines an opening **22** at a distal end **24** thereof dimensioned to allow the surgical object (not shown) to pass therethrough, thereby facilitating percutaneous access to a patient's internal cavity with the surgical object.

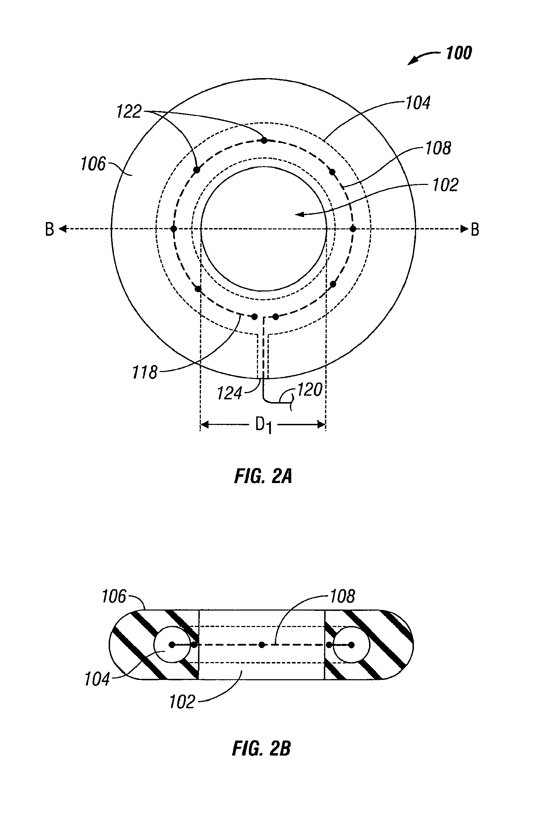

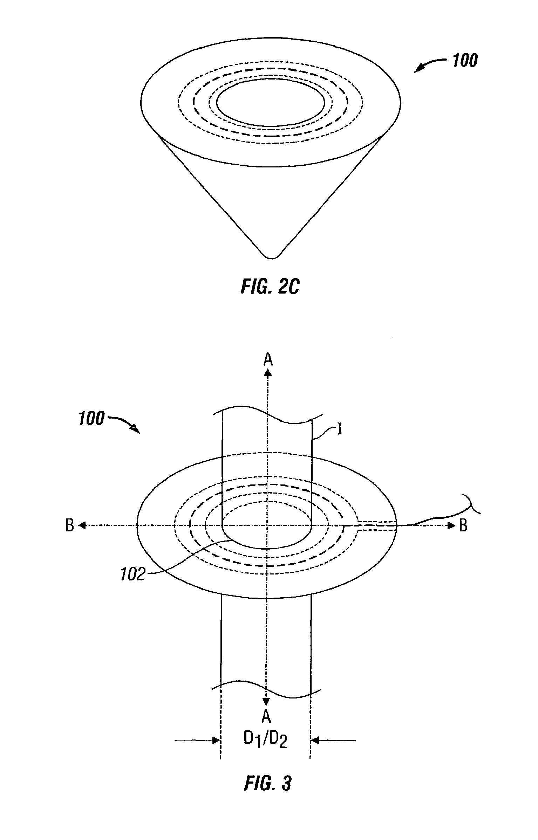

Referring now to **FIGS. 2A-5****,** seal member **100** includes an aperture **102** that extends therethrough and is dimensioned to removably receive the surgical object **"I"** (**FIG. 3**) such that a substantially fluid-tight seal may be subsequently formed therewith. In one embodiment, seal member **100** may define a substantially torroidal configuration (**FIGS. 2A-2B** and **FIGS. 3-5****),** whereas in an alternate embodiment, seal member **100** may define a substantially conical configuration **(****FIG. 2C****)** that extends distally to thereby facilitate the insertion of surgical object "**I**" **(****FIG. 3****)** within seal member **100.**

Seal member **100** further includes an internal channel **104** formed beneath a proximal surface **106** thereof. Channel **104** is adapted to receive a closure member **108** (or a portion thereof) and is disposed substantially adjacent aperture **102.** Channel **104** defines an annular configuration and may be created during the formation of seal member **100,** or subsequently thereafter, through any suitable method, including but not being limited to drilling, milling, or molding.

Seal member **100** may be formed of any suitable biocompatible and deformable material, including but not being limited to elastomeric materials such as natural rubber, synthetic polyisoprene, butyl rubber, halogenated butyl rubbers, polybutadiene, styrenebutadiene rubber, nitrile rubber, hydrogenated nitrile rubbers, chloroprene rubber, ethylene propylene rubber, ethylene propylene diene rubber, epichlorohydrin rubber, polyacrylic rubber, silicone rubber, fluorsilicone rubber, fluoroelastomers, perfluoroelastomers, polyether block amides, chlorosulfonated polyethylene, ethylene-vinyl acetate, thermoplastic elastomers, thermoplastic vulcanizers, thermoplastic polyurethane, thermoplastic olefins, resilin, elastin, and polysulfide rubber. Forming seal member **100** from such a material allows the seal member **100** to reversibly transition between a first (unbiased) condition **(****FIGS. 2A-2B****)** and a second (constricted) condition **(****FIG. 3****).** In the first condition, aperture **102** defines a first dimension **"D₁"** measured along an axis **"B-B"** that is transverse in relation to the longitudinal axis **"A-A".** The first dimension **"D₁"** is appreciably larger than an outer dimension "**D_{I}**" of surgical object "**I**" such that surgical object "**I**" may be inserted into aperture **102** with little resistance. While the use of a surgical object "**I**" with an outer dimension lying generally within the range of approximately 5mm to approximately 15mm is conventional, the employ of substantially larger and smaller surgical objects in connection with surgical access assembly **10 (****FIG. 1****)** is not beyond the scope of the present disclosure.

In the second condition, aperture **102** defines a second transverse dimension "**D₂**" that substantially approximates the outer diameter **"D_{I}"** of surgical object **"I"** such that a substantially fluid-tight seal is formed therewith, thereby curtailing the escape of insufflation gas through seal member **100** when surgical object **"I"** is inserted therethrough. Forming seal member **100** of a deformable material allows seal member **100,** and consequently aperture **102,** to substantially conform to the dimensions of surgical object **"I"** subsequent to the insertion thereof, thereby enhancing the quality of the fluid-tight sealed formed with surgical object "**I**".

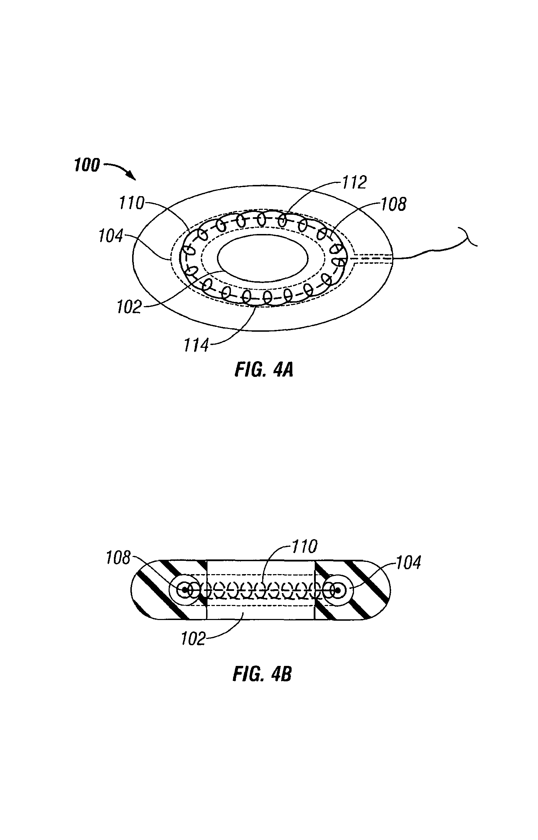

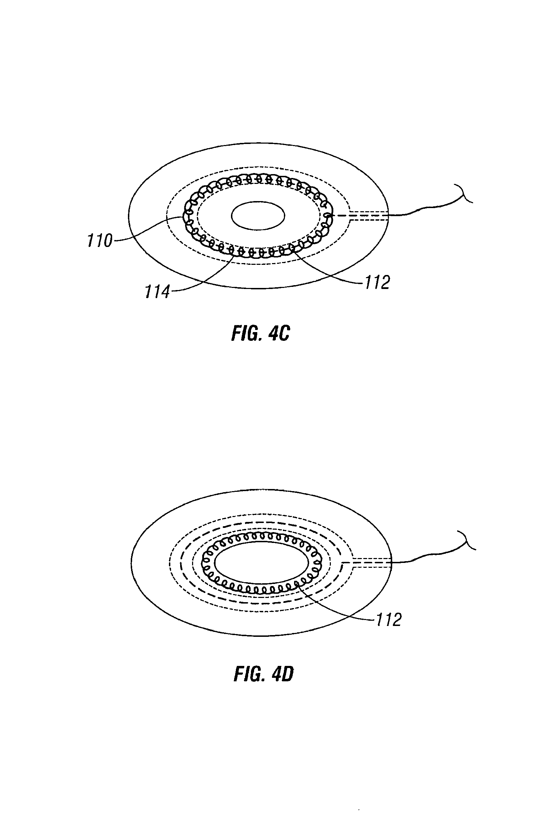

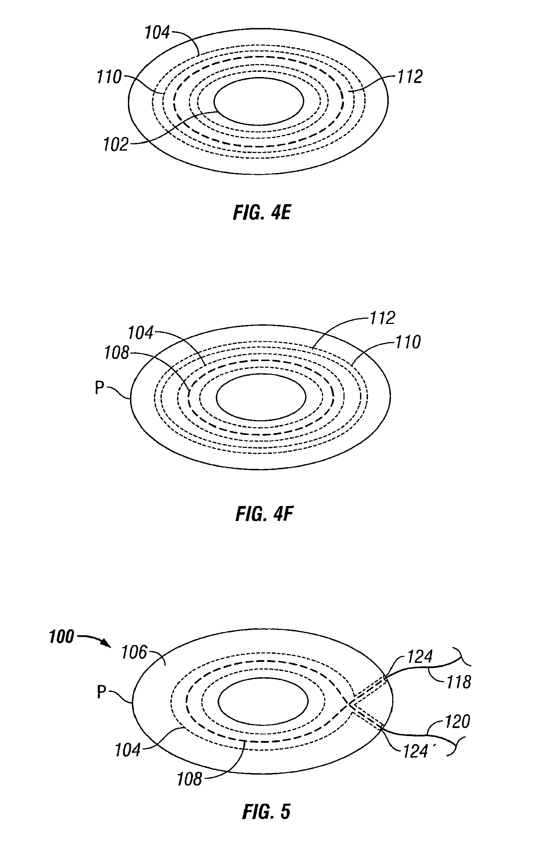

Seal member **100** is adapted to be normally biased towards the first condition. In one embodiment, this is accomplished by forming seal member **100** of a material that is at least semi-resilient in nature. Alternatively, or additionally, the resiliency of seal member **100** may be achieved, or augmented, through the incorporation of one or more biasing members **110.** As seen in **FIGS. 4A-4F****,** biasing member **110** defines a passage **112** therethrough which may accommodate closure member **108.** Biasing member **110** may be disposed within channel **104 (****FIGS. 4A-4C** **and** **FIG. 4E****),** between channel **104** and aperture **102 (****FIG. 4D****),** or between channel **104** and a periphery **"P"** of seal member **100 (****FIG. 4F****).** Biasing member **110** may be formed of any material adapted to resiliently transition between a normal condition **(****FIG. 4A****)** and a deformed condition **(****FIG. 4C****)** upon the application of a force thereto and the removal of the force therefrom, respectively. Although biasing member **110** is depicted as a spring **114** in the embodiment of **FIGS. 4A-4D****,** additional adaptations of biasing member **110,** such as a hollow tubular structure **(****FIGS. 4E-4F****),** are also within the scope of the present disclosure.

Referring now to **FIGS. 2A** and **5**, closure member **108** is an elongated member having respective first and second ends **118, 120.** Closure member **108** is at least partially disposed within internal channel **104,** as previously described, and acts to facilitate the transition of seal member **100** from the first condition to the second condition, which is discussed herein below. Closure member **108** may be any elongated member, such as a suture, ligature, cable, or the like, formed of a biocompatible material, e.g., stainless steel, fiber, or polymers.

As seen in **FIG. 2A****,** in one embodiment, first end **118** of closure member 108 is secured within seal member **100** at one or more locations **122** within channel **104,** whereas second end **120** is free, extending through channel **104,** through an egress **124** in communication therewith, and from seal member **100.** As seen in **FIG. 5****,** in an alternate embodiment, the respective first and second ends **118, 120** of closure member **108** are each free, extending through channel **104** and from seal member **100** through corresponding egresses **124, 124'** such that the respective first and second ends **118, 120** of closure member **108** cross one another. Egress **124,** or egresses **124, 124',** may be formed in any suitable surface of seal member **100,** including but not being limited to proximal surface **106** or a periphery **"P"** thereof. While **FIGS. 1-5** depict seal member **100** as including only a single closure member **108,** the incorporation of one or more additional closure members is not beyond the scope of the present disclosure.

Referring now to **FIGS. 1****, 2A-2B,** and **3,** the use and function of surgical access assembly **10** will be discussed. Initially, the target work site is insufflated with a suitable biocompatible gas, e.g., CO₂ gas, such that a larger internal work space may be created within the patient, thereby providing greater access to internal organs, cavities, tissues, etc. The insufflation may be performed with an insufflation needle or similar device, as is conventional in the art. Following insufflation, access member **16** is positioned within percutaneous access point **18** formed in the patient's tissue **"T",** which may be either preexisting or created by the clinician using an obturator (not shown) or the like, as is known in the art. Subsequently, surgical object **"I"** is introduced to surgical access assembly **10** through housing **14.** As seen in **FIG 1****,** housing **14** includes an opening **26** configured to permit closure member **108** to pass therethrough such that a portion of closure member **108** is disposed externally of surgical access assembly **10.** This allows the clinician to apply tension to closure member **108** by applying a force thereto, in the direction of arrow **"X"** for example, e.g., by grasping and drawing closure member **108** away from surgical access assembly **10.**

Prior to the application of any force to closure member **108,** seal member **100** is in the first condition **(****FIGS. 2A-2B****)** such that surgical object **"I" (****FIG. 3****)** may be freely inserted into, and passed through, aperture **102** with relatively little resistance. However, as closure member **108** experiences an increase in tension, seal member **100** transitions from the first condition to the second condition **(****FIG. 3****),** in which seal member **100** is in substantial abutment with surgical object **"I"** such that a substantially fluid-tight seal is formed therewith thereby preventing the escape of insufflation gas through seal member **100.** The second condition of seal member **100,** and accordingly, the seal with surgical object **"I",** may be maintained by sustaining the tension in securing closure member **108.** To sustain the tension, closure member **108** may be held, e.g., by the clinician or another party, or closure member **108** may be secured to any suitable surface. In one embodiment, as seen in **FIG. 1****,** surgical access assembly **10** includes attachment structure **28,** which may be disposed at any suitable location thereon, including but not being limited to housing **14** or access member **16.** Attachment structure **28** is adapted to releasably engage closure member **108,** such as by tying closure member **108** thereabout, and may be configured in any manner suitable for this intended purpose.

Following the transition of the seal member **100** from the first condition to the second condition, the remainder of the surgical procedure may then be carried out through surgical access assembly **10.** Thereafter, the tension placed on closure member **108** may be relieved, such as by untying closure member **108** from attachment structure **28** in the embodiment of **FIG. 1****.** As the tension on closure member **108** is relieved, seal member **100** returns to its first condition by the bias created through the incorporation of the at least semi-resilient material into seal member **100,** the influence of biasing member **110 (****FIGS. 4A-4F****),** or both. During this transition, aperture **102** is enlarged, thereby facilitating the removal of surgical object "I" from seal member **100** will little resistance. Surgical access assembly **10** may then be removed from the access point **18** in the patient's tissue **"T",** and the access point **18** may be closed.

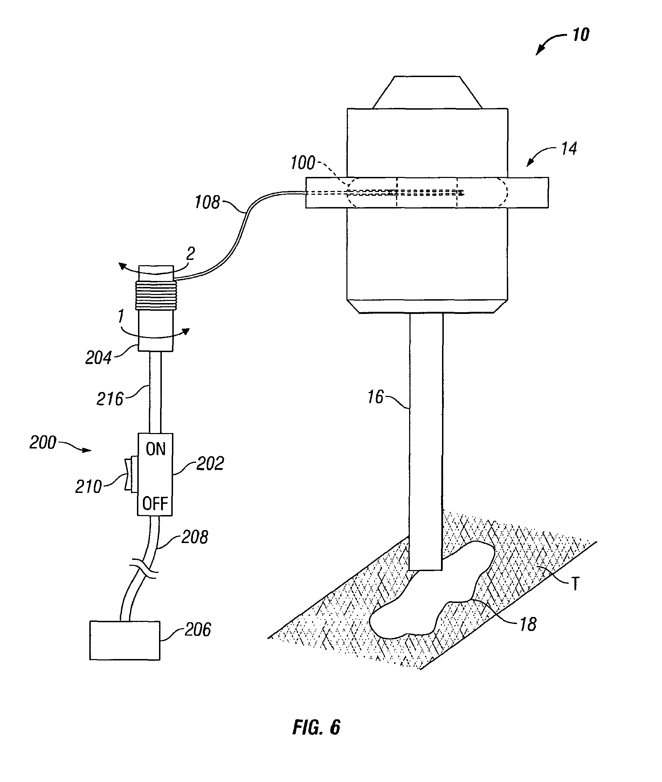

Referring now to **FIG. 6****,** in an alternate aspect of the present disclosure, surgical access assembly **10** may further include a tensioning mechanism **200.** Tensioning mechanism **200** may be used to facilitate the transition of seal member **100** from the first condition to the second condition in lieu of manually pulling or drawing upon closure member **108.** Tensioning mechanism **200** includes a motor **202** operatively connected to a spool member **204.**

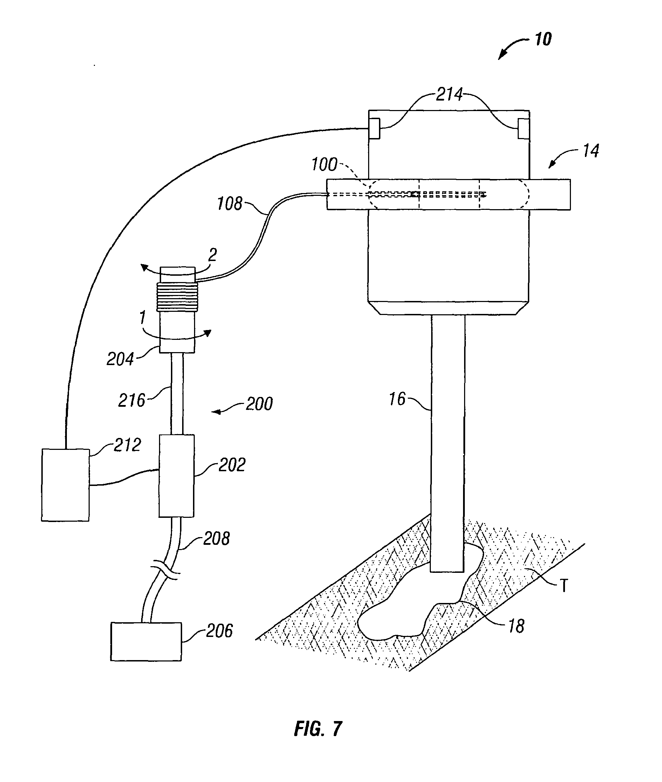

Motor **202** is connected to a power source **206** through a cable or wire **208** such that electrical energy may be communicated thereto, thereby facilitating the selective actuation of motor **202.** Motor **202** may be any mechanism suitable for the intended purpose of translating an electrical signal or current into mechanical output. In one embodiment, motor **202** may be actuated by the clinician through the use of an "on/off" mechanism, such as a switch **210.** As seen in **FIG. 7****,** in another embodiment, however, the actuation of motor **202** is controlled by a processing unit **212.** In this embodiment, processing unit **212** is in communication with motor **202** and sensing means **214,** e.g., through the exchange of electrical signals. One or more sensors **214** may be disposed within housing **14,** or at any other suitable location within or along surgical access assembly **10,** that are responsive to one or more attributes of surgical object **"I",** including but not being limited to its color, electrical impedance, magnetic impedance, or outer dimension **"D_{I}",** such that sensor **214** may be employed to detect the presence of surgical object "I" upon its introduction to housing **14.** Upon detecting surgical object **"I",** sensor **214** generates a first electrical signal that is communicated to processing unit **212.** Processing unit **212** subsequently interprets the first electrical signal, through the employ of a logic circuit (not shown), and generates a second electrical signal that is communicated to motor **202.** The second electrical signal effectuates the actuation, and subsequent control of motor **202,** thereby regulating the tension applied to closure member **108,** as discussed in further detail herein below.

In the embodiment of **FIG. 7****,** surgical access assembly **10** may further include a delay mechanism (not shown) to provide a specified interval of time between the detection of surgical object **"I"** by sensor **214** and the subsequent actuation of motor **202.**

Motor **202** is connected to a spool member **204** by a shaft **216** such that spool member **204** is moved through a plurality of positions upon the actuation of motor **202.** In one embodiment, as seen in **FIG. 6****,** motor **202** is adapted to rotate shaft **216,** and correspondingly, spool member **204,** in first and second directions, respectively indicated by arrows **1** and **2.** Spool member **204** is secured to closure member **108,** either releasably or fixed, through any suitable means, including but not being limited to tying closure member **108** about spool member **204** or through the use of an adhesive. Accordingly, as spool member **204** is moved, e.g., in the first direction **(****FIG. 1****),** closure member **108** will be wound about spool member **204,** thereby increasing the tension in closure member **108** and facilitating the transition of seal member **100** from the first condition to the second condition. Subsequently, or intermittently therewith, closure member **108** may be unwound from spool member **204,** thereby decreasing the tension in closure member, e.g., by rotating spool member **204** in the opposite or second direction. As discussed above, seal member **100** is biased towards the first condition, and consequently, decreasing the tension in closure member **108** facilitates the return of seal member **100** to the first condition.

Referring now to **FIGS. 2A-2B****, 3,** and **6-7,** the use and function of surgical access assembly **10** in conjunction with tensioning mechanism **200** will be discussed during the course of a minimally invasive procedure. Initially, the target work site is insufflated, access member **16** is positioned within a percutaneous access point **18** formed in the patient's tissue "**T**", and surgical object **"I"** is introduced to surgical access assembly **10** through housing **14,** as discussed above with respect to the embodiments of **FIGS. 1****, 2A-2B,** and **3.**

Prior to the actuation of tensioning mechanism **200,** seal member **100** is in the first condition such that surgical object **"I"** may be inserted into, and passed through, aperture **102** with relatively little resistance. To transition seal member **100** from the first condition to the second condition, the clinician actuates tensioning mechanism **200.** It should be noted that the actuation of tensioning mechanism **200** may occur at any suitable time prior to, subsequent to, or concomitant with the insertion of surgical object **"I"** into seal member **100.**

With respect to the embodiment of **FIG. 6****,** tensioning mechanism **200** may be actuated by engaging switch **210** of motor **202,** i.e., by moving switch **210** from the "off" position to the "on" position. Alternatively, with respect to **FIGS. 7****,** motor **202** may be automatically actuated subsequent to the detection of surgical object **"I"** by sensing means **214.**

Upon the actuation of tensioning mechanism **200,** motor **202** begins to move spool member **204** through its plurality of positions, thereby winding and unwinding closure member **108** about spool member **204** and selectively increasing and decreasing the tension therein to transition seal member **100** to the second condition **(****FIG. 3****)** and thereby form a substantially fluid-tight seal with surgical object **"I".** The ability to selectively increase and decrease the tension in closure member **108** provides the clinician with a corresponding ability to regulate the second transverse dimension of aperture **102** in the second condition, thereby allowing surgical access assembly **10** to accommodate the use of surgical instruments that vary in size. The tension in closure member **108,** and accordingly, the seal formed between seal member **100** and surgical object **"I",** may be maintained throughout the duration of the surgical procedure and until such time that closure member **108** is unwound from spool member **204.** Thereafter, as the tension in closure member **108** is relieved, seal member **100** returns to its first condition by the bias created through the incorporation of a semi-resilient material into seal member **100,** the influence of biasing member **110 (****FIGS. 4A-4F****),** or both. During this transition, aperture **102** is enlarged, thereby facilitating the removal of surgical object **"I"** from seal member **100** will little resistance. Surgical access assembly **10** may then be removed from the access point **18** in the patient's tissue **"T",** and the access point **18** may be closed.

Although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to those precise embodiments, and that various other changes and modifications may be effected therein by one skilled in the art without departing from the scope or spirit of the disclosure.

## Claims

1. A surgical access assembly, which comprises:
an access member dimensioned for positioning with a patient's tissue and defining a passageway extending longitudinally therethrough configured to removably receive a surgical object;
a seal member including an internal channel defined therein and having an aperture extending therethrough, the seal member being selectively adaptable to transition between a first condition wherein the aperture includes a first diameter and a second condition wherein the aperture includes a second diameter that is smaller than the first diameter; and
at least one closure member at least partially disposed within the internal channel which is actuatable to transition the diameter of the seal member from the first condition to the second condition upon actuation thereof.

2. The surgical access assembly of claim 1, wherein the aperture defines a first transverse dimension when the seal member is in the first condition allowing for the insertion of a surgical object and the aperture defines a second transverse dimension when the closure member is actuated to move the seal member to the second condition, the second transverse dimension substantially approximating an outer dimension of the surgical object such that a substantially fluid-tight seal is formed therewith.

3. The surgical access assembly of claim 2, wherein the seal member is formed of a material that is at least semi-resilient such that the aperture of the seal member is normally biased towards the first condition.

4. The surgical access assembly of claim 1, further including at least one biasing member disposed within the seal member, the biasing member being adapted to normally bias the aperture of the seal member towards the first condition.

5. The surgical access assembly of claim 4, wherein the at least one biasing member is disposed within the internal channel.

6. The surgical access assembly of claim 5, wherein the at least one biasing member defines a passage therethrough configured to receive the at least one closure member.

7. The surgical access assembly of claim 6, wherein the biasing member is a spring.

8. The surgical access assembly of claim 1, wherein the at least one closure member extends from the seal member through an egress formed therein.

9. The surgical access assembly of claim 8, wherein the egress is formed in a proximal surface of the seal member; or the surgical access assembly of claim 8, wherein the egress is formed in a periphery of the seal member; or the surgical access assembly of claim 8, further including a housing at a proximal end thereof, the access member extending distally from the housing.

10. The surgical access assembly of claim 9, wherein the housing includes at least one opening configured to allow the at least one closure member to pass therethrough such that at least a portion of the at least one closure member is disposed externally of the surgical access assembly.

11. The surgical access assembly of claim 10, further including attachment structure configured to releasably secure the at least one closure member when the seal member is in the second condition; or the surgical access assembly of claim 10, wherein the at least one closure member is secured to a tensioning mechanism; preferably wherein the tensioning mechanism includes a motor operatively secured to the at least one closure member, the motor being selectively actuable.

12. The surgical access assembly of claim 11, wherein the at least one closure member is secured to a spool member operatively connected to the motor, the motor being adapted to reposition the spool member to thereby wind and unwind the at least one closure member about the spool member such that the tension applied to the least one closure member may be respectively increased and decreased.

13. The surgical access assembly of claim 12, wherein the tensioning mechanism includes a sensor operably coupled to the housing, the sensor being adapted to detect at least one attribute of the surgical object upon the introduction thereof into the surgical access assembly and generate a first electrical signal in response thereto to actuate the closure member.

14. The surgical access assembly of claim 13, wherein the at least one attribute of the surgical object is selected from the group consisting of an outer dimension, color, electrical impedance, and magnetic impedance.
